# EUROPÄISCHE PATENTANMELDUNG

(11) **EP 4 763 109 A1**
(43) Veröffentlichungstag der Anmeldung: **24.06.2026**
(21) Anmeldenummer: 25222839.0
(22) Anmeldetag: 12.12.2025
(51) Int. Cl.: A61B 17/29, A61B 90/70

(54) **ADAPTERVORRICHTUNG, ZERLEGBARES MEDIZINISCHES SCHAFTINSTRUMENT UND VERFAHREN ZUM REINIGEN DES ZERLEGBAREN MEDIZINISCHEN SCHAFTINSTRUMENTS UND MONTAGEVERFAHREN**

(30) Priorität: 20.12.2024 DE 102024139321
(71) Anmelder: Karl Storz SE & Co. KG, 78532 Tuttlingen (DE)
(72) Erfinder: VATTHEUER, Monja, 78532 Tuttlingen (DE); KÄRCHER, Daniel, 78532 Tuttlingen (DE); MERZ, Robin, 78532 Tuttlingen (DE); KRAMER, Claus, 78532 Tuttlingen (DE); EGLE, Michael, 78532 Tuttlingen (DE)

(57) **Zusammenfassung**

Die vorliegende Erfindung stellt eine Adaptervorrichtung und ein damit ausgestattetes zerlegbares medizinisches Schaftinstrument, ein Verfahren zum Reinigen des zerlegbaren medizinischen Schaftinstruments und ein Verfahren zum Montieren der Adaptervorrichtung zur Bereitstellung des zerlegbaren medizinischen Schaftinstruments bereit. Die Adaptervorrichtung (10) ist zur Anordnung in einem zerlegbaren medizinischen Schaftinstrument (1) mit einem Schaft (2), einer im Schaft (2) bewegbaren Übertragungsstange (40) und einer Handhabungseinrichtung (3) mit einem Gehäuseteil (30) und einem Betätigungsteil (31), das gegenüber dem Gehäuseteil (30) bewegbar ist, ausgebildet. Die Adaptervorrichtung (10) hat einen Übertragungsadapter (5), der zur lösbaren Kopplung mit dem Betätigungsteil (31) und mit der Übertragungsstange (40) ausgebildet ist, und eine Adapterhülse (6), die zur lösbaren Kopplung mit dem Schaft (2) und mit dem Gehäuseteil (30) ausgebildet ist. Die Adapterhülse (6) hat eine Axialbohrung (64, 65, 66, 67), in der ein distaler Kopplungsabschnitt (52) des Übertragungsadapters (5) angeordnet ist, und wobei der Übertragungsadapter (5) relativ zur Adapterhülse (6) translatorisch bewegbar ist. Erfindungsgemäß ist ein proximaler Abschnitt (67) der Axialbohrung (64, 65, 66, 67) der Adapterhülse (6) kegelstumpfförmig ausgebildet und weitet sich zum proximalen Ende der Axialbohrung (64, 65, 66, 67) auf, wobei der kegelstumpfförmige proximale Abschnitt (67) zur lösbaren Aufnahme einer konusförmigen Düse (21) einer Spülvorrichtung (20) ausgebildet ist.

## Beschreibung

Die Erfindung betrifft eine Adaptervorrichtung und ein damit ausgestattetes zerlegbares medizinisches Schaftinstrument. Ferner betrifft die Erfindung ein Verfahren zum Reinigen des zerlegbaren medizinischen Schaftinstruments und ein Verfahren zum Montieren der Adaptervorrichtung zur Bereitstellung des zerlegbaren medizinischen Schaftinstruments.

Aus dem Stand der Technik ist bekannt, dass medizinische, insbesondere chirurgische Instrumente für die Wiederverwendung zur Reinigung und Sterilisierung zerlegbar gestaltet werden. Ein übliches Schaftinstrument weist einen Schaft, eine Handhabungseinrichtung an dem proximalen Ende des Schafts und einen Werkzeugeinsatz mit einem Werkzeug am distalen Ende des Schafts und einer Übertragungsstange auf, die sich zur Kopplung des Werkzeugs mit der Handhabungseinrichtung durch den Schaft erstreckt. Bei einem zerlegbaren Schaftinstrument kann üblicherweise der Schaft von der Handhabungseinrichtung und ggf. auch von dem Werkzeugeinsatz getrennt werden. Die Handhabungseinrichtung weist ein mit dem Schaft verbundenes Gehäuseteil und ein relativ dazu bewegliches Betätigungsteil auf, das mit der Übertragungsstange gekoppelt ist, sodass eine Relativbewegung zwischen dem Betätigungsteil und dem Gehäuseteil der Handhabungseinrichtung eine translatorische Bewegung der Übertragungsstange im Schaft zur Betätigung des Werkzeugs am distalen Schaftende bewirkt.

Dabei weist die Übertragungsstange an ihrem proximalen Ende ein Kopplungselement zur lösbaren Kopplung mit dem Betätigungsteil der Handhabungseinrichtung auf, sodass die Übertragungsstange von der Handhabungseinrichtung getrennt, und, wenn ferner der Werkzeugeinsatz von dem Schaft trennbar ist, nach distal aus dem Schaft herausgezogen werden kann. Da Schaftdurchmesser und Querschnitt der Übertragungsstange aufeinander abgestimmt sind, darf bei zerlegbaren Schaftinstrumenten dann die Größe des Kupplungselements den Querschnitt der Übertragungsstange nicht übersteigen. Allerdings weisen Schaftinstrumente für minimal- und mikroinvasive Eingriffe möglichst dünne Schäfte auf, sodass der Querschnitt der Übertragungsstangen und damit auch die Größe der Kupplungselemente entsprechend klein dimensioniert werden müssen. Allerdings bestimmt die Größe des Kupplungselements die von dem Betätigungsteil auf die Übertragungsstange übertragbare Betätigungskraft. Daher müssen für unterschiedliche Schaftdurchmesser unterschiedliche Handhabungseinrichtungen oder Handhabungseinrichtungen mit Vorrichtungen verwendet werden, die entsprechende Anpassungen an unterschiedliche Schaftdurchmesser und unterschiedlich große Kopplungselemente gestatten.

DE 10 2012 200 073 A1 beschreibt ein medizinisches Instrument, das eine Kombination unterschiedlicher Schäfte und unterschiedlicher Übertragungsstangen mit einer Handhabungseinrichtung ermöglicht. Dazu ist ein Übertragungsadapter vorgesehen, der einerseits mit dem Kupplungselement am proximalen Ende der Übertragungsstange und andererseits mit dem Betätigungsteil der Handhabungseinrichtung gekoppelt wird. Zur Kopplung mit der Übertragungsstange weist der Übertragungsadapter gelenkig schwenkbare Greifbacken auf, die durch ein elastisches Element in eine Position vorgespannt werden, in der die Greifbacken das Kupplungselement der Übertragungsstange form- und kraftschlüssigen halten. Der Übertragungsadapter ist bewegbar in einem hülsenartigen Schaftadapter angeordnet, der einerseits mit dem Gehäuseteil der Handhabungseinrichtung und andererseits dem Schaft lösbar gekoppelt ist.

Durch den Übertragungsadapter und den Schaftadapter lassen sich ein dünner Schaft mit einem Durchmesser kleiner als 5 mm, z. B. 3,5 mm, und ein Werkzeugeinsatz mit entsprechend dünner Übertragungsstange mit einer herkömmlichen Handhabungseinrichtung koppeln. Nachteilig werden dabei aber viele Bauteile und ein entsprechender Montageaufwand benötigt. Denn bei der Aufbereitung des zerlegten Instruments muss zum Spülen des dünnen Schafts ein Spülanschluss integriert werden, der in üblicher Weise am proximalen Schaftende an der Schnittstelle zur Handhabungseinrichtung angeordnet wird. Ein axiales Spülen von der proximalen Seite des Übertragungsadapters aus ist nicht möglich, da aufgrund der für die gelenkigen Greifbacken erforderlichen offenen Bauweise des Übertragungsadapters der am distalen Ende des Schafts ankommende Spüldruck nicht für eine zuverlässige und sichere Aufbereitung ausreicht.

DE 10 2016 118 304 A1 offenbart eine Sicherheitseinrichtung zur Verhinderung von Überlastung der Mechanik bei mikroinvasiven Instrumenten. Sie umfasst dazu eine in dem Schaft bewegbare Übertragungseinrichtung mit Anschlagflächen zur Begrenzung der Bewegung dieser Übertragungseinrichtung.

DE 10 2022 107 371 A1 zeigt eine Schaftbaugruppe mit rohrförmigem Schaft und beweglichen Arbeitselementen sowie einer Griffbaugruppe mit Betätigungselement, die reversibel lösbar und um die Längsachse verdrehbar miteinander verbunden sind. Über einen axial angeordneten Spülanschluss kann Spülflüssigkeit in den Ringspalt zwischen Schaft und Stangenelement eingeleitet werden. Die Griffbaugruppe besitzt einen Kopf mit distal und proximal angeordneten Teilen, wobei der Schaft drehfest am distalen Kopfteil befestigt ist und beide Kopfteile durch zentrale Öffnungen und Bohrungen axial durchsetzt sind.

DE 20 2021 102 135 U1 offenbart ein chirurgisches Instrument mit einem rohrförmigen Schaft mit mindestens einem beweglichen Arbeitselement, das über ein verschiebbares Stangenelement durch Betätigung eines Griff-Betätigungselements bewegt wird. Es besitzt einen axial angeordneten Spülanschluss, um Spülflüssigkeit in den Ringraum zwischen Schaft und Stangenelement einzuleiten.

US 2017/0367722 A1 (DE 10 2012 022 573 A1) lehrt ein medizinisches Instrument, dessen Schaft und Handhabungseinrichtung für Reinigung und Sterilisation zerlegbar sind und bei dem unterschiedliche Übertragungseinrichtungen mit einer Standard-Handhabungseinrichtung kombiniert werden können. Kern der Lösung ist ein Übertragungsadapter, der zwischen der Handhabungseinrichtung und dem proximalen Ende der Übertragungseinrichtung angeordnet wird und durch zwei Kupplungen eine lösbare mechanische Verbindung ermöglicht. Dadurch wird die Anpassung an verschiedene Schaftdurchmesser und Kupplungsgrößen erleichtert.

Ausgehend von diesem Stand der Technik ist es Aufgabe der vorliegenden Erfindung, ein verbessertes Adapterkonzept bereitzustellen.

Diese Aufgabe wird durch eine Adaptervorrichtung mit den Merkmalen des Anspruchs 1 gelöst.

Ausführungsbeispiele der vorliegenden Erfindung beruhen auf der Idee, einen Übertragungsadapter vorzusehen, der einerseits mit dem proximalen Ende einer Übertragungsstange in einem Schaft für ein medizinisches Schaftinstrument und andererseits mit einer Handhabungseinrichtung für ein medizinisches Schaftinstrument koppelbar ist. Insbesondere ist für eine Mehrzahl unterschiedlicher Übertragungsstangen bzw. für eine Mehrzahl von Übertragungsstangen mit unterschiedlich ausgebildeten Kupplungen an ihren proximalen Enden jeweils ein Übertragungsadapter vorgesehen. Ein distales Ende bzw. eine Kupplung am distalen Ende der Übertragungsstange ist jeweils an eine bestimmte Ausgestaltung des proximalen Endes einer Übertragungsstange angepasst, die proximalen Enden der Übertragungsadapter sind gleich und insbesondere an eine Standard-Handhabungseinrichtung angepasst.

Ein Übertragungsadapter für ein medizinisches Instrument mit einem Schaft, einer im Schaft bewegbaren Übertragungsstange und einer Handhabungseinrichtung mit einem Gehäuseteil, das mit einem proximalen Ende des Schafts mechanisch starr koppelbar ist, und einem Betätigungsteil, das gegenüber dem Gehäuseteil bewegbar ist, umfasst eine erste Kupplung zur lösbaren mechanischen Kopplung mit dem Betätigungsteil der Handhabungseinrichtung und eine zweite Kupplung zur lösbaren oder unlösbaren mechanischen Kopplung mit einem proximalen Ende der Übertragungsstange.

Der Übertragungsadapter ist insbesondere Bestandteil eines medizinischen Schaftinstruments oder mit anderen Bestandteilen zusammen zur Bildung eines medizinischen Schaftinstruments vorgesehen und ausgebildet. Der Schaft ist gerade oder gekrümmt, teilweise oder vollständig starr oder teilweise oder vollständig elastisch oder plastisch verformbar. Die Übertragungsstange ist im Schaft translatorisch und / oder rotatorisch bewegbar, um eine Zug- und / oder Druckkraft und / oder ein Drehmoment zwischen der Handhabungseinrichtung und einem distalen Ende des Schafts, insbesondere einem Werkzeug am distalen Ende des Schafts, zu übertragen.

Die weitere Aufgabe, ein verbessertes zerlegbares medizinisches Schaftinstrument bereitzustellen, wird durch das zerlegbare medizinische Schaftinstrument mit den Merkmalen des unabhängigen Anspruchs 10 gelöst.

Eine weitere Aufgabe besteht darin, das Reinigen eines zerlegbaren medizinisches Schaftinstruments zu verbessern, und wird durch das Verfahren mit den Merkmalen des unabhängigen Anspruchs 11 gelöst.

Eine noch weitere Aufgabe, die Montage der Adaptervorrichtung in einem zerlegbaren medizinischen Schaftinstrument, wird durch das Verfahren mit den Merkmalen des unabhängigen Anspruchs 12 gelöst.

Weiterbildungen bzw. bevorzugte Ausführungsformen der Adaptervorrichtung, des Schaftinstruments, des Reinigungsverfahrens und des Montageverfahrens sind in den jeweiligen Unteransprüchen ausgeführt.

Eine erfindungsgemäße Adaptervorrichtung ist ausgebildet zur Anordnung in einem zerlegbaren medizinischen Schaftinstrument, das einen Schaft, eine im Schaft bewegbaren Übertragungsstange und eine Handhabungseinrichtung mit einem Gehäuseteil und einem Betätigungsteil aufweist, das gegenüber dem Gehäuseteil bewegbar ist. Gemäß einer ersten Ausführungsform weist die Adaptervorrichtung einen Übertragungsadapter, der zur lösbaren Kopplung mit dem Betätigungsteil und mit der Übertragungsstange ausgebildet ist, und eine Adapterhülse auf, die zur lösbaren oder unlösbaren Kopplung mit dem Schaft und mit dem Gehäuseteil ausgebildet ist. Dabei weist die Adapterhülse eine Axialbohrung auf, in der ein distaler Kopplungsabschnitt des Übertragungsadapters angeordnet ist. Der Übertragungsadapter ist relativ zur Adapterhülse translatorisch bewegbar ist. Erfindungsgemäß ist ein proximaler Abschnitt der Axialbohrung der Adapterhülse kegelstumpfförmig ausgebildet und weitet sich zum proximalen Ende der Axialbohrung auf, wobei der kegelstumpfförmige proximale Abschnitt, der im Folgenden kürzer auch Konusabschnitt genannt wird, zur lösbaren Aufnahme einer konusförmigen Düse einer Spülvorrichtung ausgebildet ist.

Mittels einander entsprechenden Kegelstumpfformen des Konusabschnitts und der Düse kann die Düse zur Reinigung klemmfest in den Konusabschnitt aufgenommen und so abgedichtet werden. Damit wird eine sichere Aufbereitung insbesondere auch von dünnen Schäften durch axiale Zufuhr eines Spülmediums von proximaler Seite der Adaptervorrichtung ermöglicht, sodass ausreichend Spülmedium distal am Schaft austreten kann. Dabei ist die Adaptervorrichtung mit bestehenden Instrumentenkomponenten aus der Serienfertigung wie Handhabungseinrichtungen, Übertragungsstangen, und Schäfte insbesondere auch mit einem Durchmesser kleiner als 5 mm, z. B. 3,5 mm, kompatibel und weist einen einfachen Aufbau hinsichtlich Fertigung und Montage auf.

Die erfindungsgemäße Adaptervorrichtung umfasst zwei Adapter als mechanische Verbindungsstücke zwischen den verschiedenen Schnittstellen der Instrumentenkomponenten, wenn die vorliegenden Kopplungselemente unterschiedlich dimensioniert sind. D. h., dass ein distales Kopplungselement des Übertragungsadapters zur lösbaren Kopplung mit dem proximalen Kopplungselement der Übertragungsstange ausgebildet ist, und ein proximales Kopplungselement des Übertragungsadapters zur lösbaren Kopplung mit einem Kopplungselement des Betätigungsteils der Handhabungseinrichtung. Die Adapterhülse stellt Kopplungselemente zur lösbaren oder unlösbaren Kopplung einerseits mit dem proximalen Ende des Schafts und andererseits mit dem Gehäuseteil der Handhabungseinrichtung bereit.

Nach einer weiteren Ausführungsform der erfindungsgemäßen Adaptervorrichtung weist ein proximaler Endabschnitt der Adapterhülse ein Außengewinde auf, das folglich den Konusabschnitt umgibt. Dieses Außengewinde ist zur lösbaren Verbindung mit einem die Düse umgebenden Innengewinderohrabschnitt der Spülvorrichtung ausgebildet, sodass beim Aufschrauben des Innengewinderohrabschnitts auf das Außengewinde der Adapterhülse die Düse der Spülvorrichtung klemmfest in dem Konusabschnitt aufgenommen wird. Die Schraubverbindung sorgt für eine sichere Abdichtung der Klemmverbindung auch bei höheren Spüldrücken.

Vorteilhaft kann nach einer weiteren Ausführungsform der erfindungsgemäßen Adaptervorrichtung vorgesehen sein, dass der proximale kegelstumpfförmige proximale Abschnitt entsprechend einem genormten Verbindungssystem geformt ist. Dabei kann das Luer-System (international standardisiert in ISO-Norm ISO 80369) besonders bevorzugt sein, dessen Anwendung im medizinischen Bereich, z. B. für Kanülen und Spritzen etc. üblich ist. Die Kegelstumpfform der männlichen und weiblichen Verbindungsstücke weist dabei einen Kegelwinkel von 3,44 ° auf. Die Steckverbindung ohne Schraubgewinde wird auch als "Luer-Slip" bezeichnet und die Steckverbindung mit Schraubgewinde als "Luer-Lock".

Eine weitere Ausführungsform der erfindungsgemäßen Adaptervorrichtung bezieht sich darauf, dass der distale Kopplungsabschnitt des Übertragungsadapters eine axiale Aufnahmebohrung und zumindest eine damit verbundene Eingriffsöffnung in Radialrichtung aufweist. Die Adaptervorrichtung weist hierbei zumindest einen Eingriffsschlitten mit einem Steckabschnitt auf, der in der Eingriffsöffnung angeordnet ist und sich in die Aufnahmebohrung des Übertragungsadapters erstreckt. Der Steckabschnitt hat eine Aussparung, die zur Aufnahme eines proximalen Kopplungselements der Übertragungsstange ausgebildet ist. Dabei weist die Adapterhülse an einem mittleren Abschnitt zumindest eine Längsöffnung auf, die mit der Axialbohrung verbunden ist und länger ist als der Steckabschnitt, der sich durch die Längsöffnung zur Anordnung in der Eingriffsöffnung des distalen Kopplungsabschnitts erstreckt. So ist der Eingriffsschlittenmit mit dem Übertragungsadapter relativ zur Adapterhülse translatorisch bewegbar.

Dabei kann der Steckabschnitt vorzugsweise zum formschlüssigen Einstecken in die Eingriffsöffnung ausgebildet sein und auch die Aussparung zur formschlüssigen Aufnahme des proximalen Kopplungselements. Ferner kann eine Breite des Steckabschnitts einer Breite der Längsöffnung entsprechen, sodass der Eingriffsschlitten bei der translatorischen Bewegung mit dem Übertragungsadapter sicher in der Längsöffnung geführt wird.

Gemäß einer vorteilhaften Ausführungsform kann der Eingriffsschlitten einen Schieberabschnitt aufweisen, von dem sich der Steckabschnitt erstreckt. Und die Adapterhülse kann an dem mittleren Abschnitt um die Längsöffnung eine Gleitfläche aufweisen, auf der der Schieberabschnitt des Eingriffsschlittens translatorisch bewegbar anliegt. Eine Länge und Breite des Schieberabschnitts sind dabei so bemessen, dass die Längsöffnung stets durch den Schieberabschnitt abgedeckt ist. Auf diese Weise dichtet der Eingriffsschlitten die Axialbohrung der Adapterhülse ab.

Vorzugsweise kann die Adaptervorrichtung nach einer weiteren Ausführungsform zwei Eingriffsschlitten, zwei Eingriffsöffnungen an dem distalen Kopplungsabschnitt des Übertragungsadapters und zwei Längsöffnungen an dem mittleren Abschnitt der Adapterhülse aufweisen, die jeweils in Radialrichtung gegenüberliegend angeordnet sind.

Zur Sicherung des oder der Eingriffsschlitten weist dieser gemäß einer weiteren Ausführungsform an einer von dem Steckabschnitt abgewandten Außenseite zumindest eine in Umfangsrichtung verlaufende Aufnahmenut auf, in der ein elastischer O-Ring aufgenommen ist, der den mittleren Abschnitt der Adapterhülse umgibt.

Ferner kann nach einer noch weiteren Ausführungsform vorgesehen sein, dass der distale Kopplungsabschnitt zur axialen Führung des Übertragungsadapters in der Adapterhülse eine Kantform mit zumindest einer axialparallelen Führungsfläche aufweist.

Zur Kopplung der Adapterhülse einer erfindungsgemäßen Adaptervorrichtung mit einem Schaft ist nach einer weiteren Ausführungsform ein distaler Abschnitt der Axialbohrung zur lösbaren Aufnahme eines proximalen Endes des Schafts ausgebildet. Der distale Abschnitt ist mit einem im Durchmesser reduzierten Verbindungsabschnitt der Axialbohrung verbunden ist, der zum Durchtritt der Übertragungsstange ausgebildet ist. Dabei kann an dem Verbindungsabschnitt benachbart zu dem distalen Abschnitt eine Dichtnut ausgeformt sein, in der ein Dichtring zur Abdichtung der Übertragungsstange angeordnet ist.

Zur weiteren Kopplung der Adapterhülse mit dem Gehäuseteil der Handhabungseinrichtung kann nach einer weiteren Ausführungsform ein distaler Endabschnitt der Adapterhülse zylindrisch geformt sein und durch einen Wellenbund begrenzt werden, der als Anschlag zur Anordnung in dem Gehäuseteil ausgebildet ist.

Ein erfindungsgemäßes zerlegbares medizinisches Schaftinstrument weist in einer ersten Ausführungsform eine Handhabungseinrichtung mit einem Gehäuseteil und einem relativ dazu bewegbaren Betätigungsteil, einen Schaft, eine im Schaft bewegbare Übertragungsstange und eine erfindungsgemäße Adaptervorrichtung auf. Die Adaptervorrichtung umfasst einen Übertragungsadapter zur lösbaren Kopplung mit dem Betätigungsteil und mit der Übertragungsstange und eine Adapterhülse zur lösbaren oder unlösbaren Kopplung mit dem Gehäuseteil und mit dem Schaft. Dabei ist ein distaler Kopplungsabschnitt des Übertragungsadapters in einer Axialbohrung der Adapterhülse angeordnet, und der Übertragungsadapter ist relativ zur Adapterhülse translatorisch bewegbar ist. Durch die Verwendung der erfindungsgemäßen Adaptervorrichtung in dem Schaftinstrument wird eine sichere Aufbereitung insbesondere auch von dünnen Schäften durch axiale Zufuhr eines Spülmediums von proximaler Seite der Adaptervorrichtung ermöglicht, sodass ausreichend Spülmedium distal am Schaft austreten kann. Die Konstruktion vereinfacht sich, wodurch das Schaftinstrument hinsichtlich Fertigung und Montage einfach aufgebaut werden kann.

Ein erfindungsgemäßes Verfahren bezieht sich auf das Reinigen eines erfindungsgemäßen zerlegbaren medizinisches Schaftinstruments, das eine erfindungsgemäße Adaptervorrichtung aufweist. Das Reinigungsverfahren umfasst die Schritte:
- Lösen der Kopplung des Übertragungsadapters mit dem Betätigungsteil und Lösen der Kopplung der Adapterhülse von dem Gehäuseteil, und
- Trennen des mit der Übertragungsstange gekoppelten Übertragungsadapters und insbesondere der mit dem Schaft gekoppelten Adapterhülse aus der Handhabungseinrichtung,
- Lösen der Kopplung des Übertragungsadapters mit der Übertragungsstange und Entfernen der Übertragungsstange aus dem Schaft,
- Anordnen der konusförmigen Düse einer Spülvorrichtung in dem kegelstumpfförmigen proximalen Abschnitt der Axialbohrung der Adapterhülse und
- Zuführen eines Spülmediums mit der Spülvorrichtung durch die Düse in die Axialbohrung der Adapterhülse und den damit gekoppelten Schaft, sodass das Spülmedium am distalen Ende des Schafts austritt, dadurch Reinigen des medizinischen Schaftinstruments.

Ein ebenfalls erfindungsgemäßes Verfahren bezieht sich auf das Montieren einer erfindungsgemäßen Adaptervorrichtung zur Bereitstellung eines erfindungsgemäßen zerlegbaren medizinischen Schaftinstruments und umfasst die Schritte:
- Bereitstellen des Übertragungsadapters und der Adapterhülse und Bereitstellen der Handhabungseinrichtung mit dem Gehäuseteil und dem relativ dazu bewegbaren Betätigungsteil und dem Schaft mit der im Schaft bewegbaren Übertragungsstange,
- Anordnen des distaler Kopplungsabschnitts des Übertragungsadapters in der Axialbohrung der Adapterhülse,
- lösbares Koppeln des Übertragungsadapters mit der Übertragungsstange und lösbar oder unlösbar Koppeln der Adapterhülse mit dem Schaft,
- Anordnen des mit der Übertragungsstange gekoppelten Übertragungsadapters und der mit dem Schaft gekoppelten Adapterhülse in der Handhabungseinrichtung, dabei
- lösbares Koppeln der Adapterhülse mit dem Gehäuseteil und lösbares Koppeln des Übertragungsadapters mit dem Betätigungsteil.

Nach einer weiteren Ausführungsform des Montageverfahrens umfasst das lösbare Koppeln des Übertragungsadapters mit der Übertragungsstange ferner die Schritte:
- beim Anordnen des distaler Kopplungsabschnitts des Übertragungsadapters in der Axialbohrung der Adapterhülse Ausrichten der zumindest einen mit der axialen Aufnahmebohrung verbundenen Eingriffsöffnung am distalen Kopplungsabschnitt mit der zumindest einen Längsöffnung an dem mittleren Abschnitt der Adapterhülse,
- Anordnen des proximalen Kopplungselements der Übertragungsstange in der Aufnahmebohrung am distalen Kopplungsabschnitt,
- Bereitstellen des zumindest eines Eingriffsschlittens,
- Einführen des Steckabschnitts des Eingriffsschlittens durch die Längsöffnung (62) und die Eingriffsöffnung in die Aufnahmebohrung und
- Aufnehmen des proximalen Kopplungselements der Übertragungsstange in der Aussparung des Steckabschnitts.

Nach noch einer weiteren Ausführungsform des Montageverfahrens umfasst das lösbare Koppeln des Übertragungsadapters mit der Übertragungsstange ferner den Schritt des Anordnens eines elastischen O-Rings in der Aufnahmenut an der Außenseite des Eingriffsschlittens.

Weitere Ausführungsformen sowie einige der Vorteile, die mit diesen und weiteren Ausführungsformen verbunden sind, werden durch die nachfolgende ausführliche Beschreibung unter Bezug auf die begleitenden Figuren deutlich und besser verständlich. Gegenstände oder Teile derselben, die im Wesentlichen gleich oder ähnlich sind, können mit denselben Bezugszeichen versehen sein. Die Figuren sind lediglich eine schematische Darstellung einer Ausführungsform der Erfindung. Die Zeichnungen, die Beschreibung und die Ansprüche enthalten zahlreiche Merkmale in Kombination. Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Dabei zeigen:
- Fig. 1: eine Seitenansicht eines zerlegbaren Schaftinstruments aus dem Stand der Technik mit radialem Spülanschluss,
- Fig. 2: eine schematische Darstellung einer Handhabungseinrichtung mit einer erfindungsgemäßen Adaptervorrichtung eines erfindungsgemäßen zerlegbaren Schaftinstruments,
- Fig. 3: eine perspektivische Explosionsansicht der erfindungsgemäßen Adaptervorrichtung,
- Fig. 4: eine Längsschnittansicht der erfindungsgemäßen Adaptervorrichtung in Kopplungsanordnung mit einem Schaft und einer Übertragungsstange,
- Fig. 5: eine Längsschnittansicht der erfindungsgemäßen Adaptervorrichtung aus Fig. 4 ohne Übertragungsstange mit Spülvorrichtung.

Die Erfindung bezieht sich auf eine Adaptervorrichtung für ein zerlegbares medizinisches Schaftinstrument und entsprechend auch auf ein damit versehenes zerlegbares medizinisches Schaftinstrument. Ferner umfasst die Erfindung Verfahren zur Reinigung des zerlegbaren medizinischen Schaftinstruments und zur Montage der Adaptervorrichtung in ein zerlegbares medizinisches Schaftinstrument.

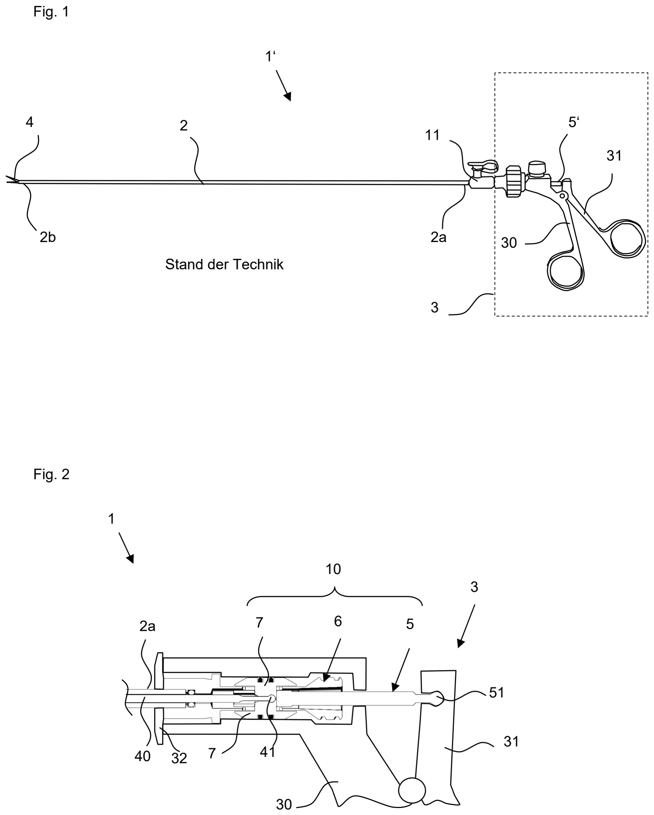

Fig. 1 zeigt ein zerlegbares medizinisches Schaftinstrument 1' aus dem Stand der Technik, das aus einer Handhabungseinrichtung 3, einem Schaft 2, und einem Werkzeugeinsatz mit einem Werkzeug 4 und einer Übertragungsstange (in Fig. 1 nicht zu sehen) zusammengesetzt ist. Das Werkzeug 4 ist am distalen Ende 2b des Schafts 2 angeordnet, sodass sich die Übertragungsstange durch den Schaft 2 bis in Handhabungseinrichtung 3 erstreckt. An seinem proximalen Ende 2a ist der Schaft 2 mit dem Gehäuseteil 30 der Handhabungseinrichtung 3 lösbar gekoppelt, die eine herkömmliche Adaptervorrichtung mit einem Übertragungsadapter 5' umfasst, der einerseits mit dem proximalen Ende der Übertragungsstange und andererseits mit dem Betätigungsteil 31 der Handhabungseinrichtung 3 lösbar gekoppelt ist, das gegenüber dem Gehäuseteil 30 beweglich ist. Durch Bewegung des Betätigungsteils 31 gegenüber dem Gehäuseteil 30 wird die über den Übertragungsadapter 5' verbundene Betätigungsstange im Schaft 2 translatorisch bewegt, um das Werkzeug 4 am distalen Ende 2b des Schafts 2 zu betätigen.

Bei dem Schaftinstrument 1' aus dem Stand der Technik ist ein Spülanschluss 11 am proximalen Ende 2a des Schafts 2 an der Schnittstelle mit der Handhabungseinrichtung 3 angeordnet. Eine anderes Spülkonzept ist aufgrund der eingangs beschriebenen offenen Bauweise des bekannten Übertragungsadapters 5' nicht möglich bzw. führt nicht zu einem ausreichenden Spülergebnis.

Ein erfindungsgemäßes zerlegbares medizinisches Schaftinstrument 1, ausschnittsweise in Fig. 2 dargestellt, unterscheidet sich von dem herkömmlichen Instrument 1' dadurch, dass es anstelle des herkömmlichen Übertragungsadapters 5' eine erfindungsgemäße Adaptervorrichtung 10 aufweist und daher auf einen Spülanschluss 11 an der Schnittstelle zwischen Schaft 2 und Handhabungseinrichtung 3 verzichten kann. Als Instrumentenkomponenten kann ein erfindungsgemäßes Schaftinstrument 1 einen Schaft 2, eine Handhabungseinrichtung 3 und ein Werkzeug 4 mit Übertragungsstange 40 wie das herkömmliche Schaftinstrument 1' aus bestehender Serienfertigung aufweisen. Hierbei ist ein erfindungsgemäßes Schaftinstrument nicht auf das in Fig. 1 beispielhaft dargestellte Zangenwerkzeug 4 beschränkt, sondern kann jegliches über eine Übertragungsstange betätigbare Werkzeug aufweisen.

Wie in Fig. 2 gezeigt, verbindet eine erfindungsgemäße Adaptervorrichtung 10 mit dem Übertragungsadapter 5 die Übertragungsstange 40 mit dem Betätigungsteil 31 und mit der Adapterhülse 6 den Schaft 2 mit dem Gehäuseteil 30, wobei die Verbindungsstellen lösbar oder unlösbar sein können. Damit ermöglicht die Adaptervorrichtung 1 die Kopplung eines Schafts 2 und einer Übertragungsstange 40 mit einer Handhabungsvorrichtung 3, wenn die Kopplungselemente der Übertragungsstange 40 und des Schafts 2 nicht mit den Kopplungselementen des Betätigungsteils 31 und des Gehäuseteils 30 korrespondieren, wie dies z. B. bei Schäften unter 5 mm und den dazu passenden Werkzeugeinsätzen bzw. Übertragungsstangen der Fall sein kann.

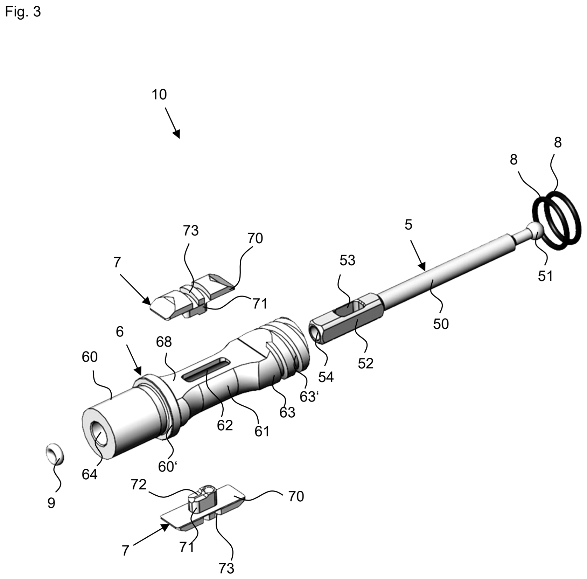

Aus Fig. 3 bis 5 wird ersichtlich, dass die Adapterhülse 6 zur Kopplung mit dem Gehäuseteil 30 einen distalen Endabschnitt 60 aufweist, der zylindrisch geformt und durch einen Wellenbund 60' begrenzt ist. Der Wellenbund 60' dient, wie in Fig. 2 angedeutet, als Anschlag zur Anordnung in dem Gehäuseteil 30, das distalseitig mit einem den Schaft 2 umschließenden Deckelelement 32 verbunden ist.

Zur weiteren Kopplung der Adapterhülse 6 mit dem Schaft 2 ist ein distaler Abschnitt 64 der Axialbohrung 64, 65, 66, 67 zur Aufnahme des proximalen Schaftendes 2a ausgebildet. Das proximale Ende 2a des Schafts 2 ist bis zu einem Anschlag aufgenommen, der durch einen Absatz zu einem im Durchmesser verringerten Verbindungsabschnitt 65 der Axialbohrung 64, 65, 66, 67 gebildet wird, der sich an den distalen Abschnitt 64 anschließt. Der Verbindungsabschnitt 65, der zum Durchtritt der Übertragungsstange 40 ausgebildet ist und in einen mittleren Abschnitt 66 der Axialbohrung 64, 65, 66, 67 mündet, weist benachbart zu dem distalen Abschnitt 64 eine Dichtnut 65' auf, in der ein Dichtring 9 zur Abdichtung der Übertragungsstange 40 angeordnet ist.

Zur Verbindung der Betätigungsstange 40, deren proximales Kopplungselement 41 einen kleineren Durchmesser als die (in Fig. 2 unbezeichnete) Kopplungsaufnahme des Betätigungsteils 31 aufweist, wird der Übertragungsadapter 5 eingesetzt. Der Übertragungsadapter 5 hat am proximalen Ende eines Stangenabschnitts 50 ein Kopplungselement 51, das in die Kopplungsaufnahme des Betätigungsteils 31 aufnehmbar ist.

Im gezeigten Beispiel ist das proximale Kopplungselement 41 der Übertragungsstange 40 und das proximale Kopplungselement 51 des Übertragungsadapters 5 jeweils kugelkopfartig ausgebildet. Allerdings sind auch Modifikationen der Adaptervorrichtung für modifizierte Schaftinstrumente denkbar, deren Kopplungselemente abweichend dazu gestaltet sind.

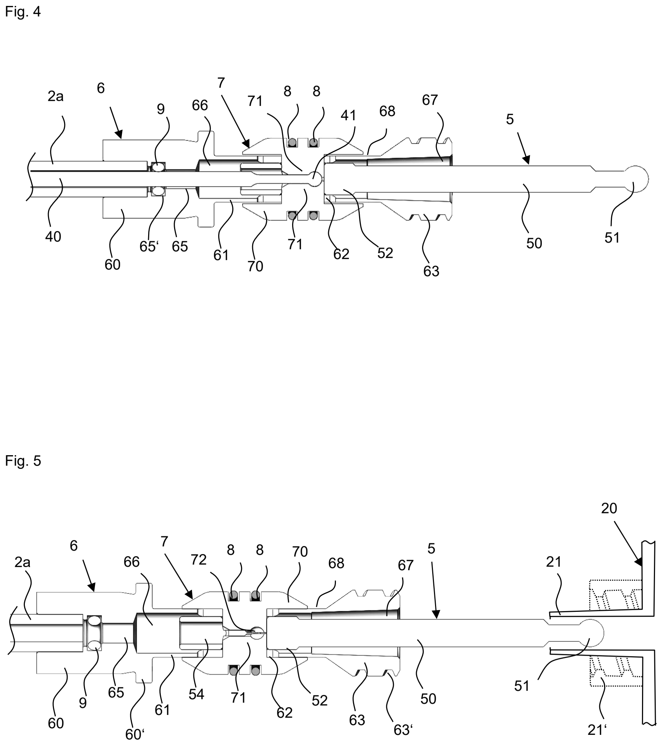

Der translatorisch bewegliche Übertragungsadapter 5 erstreckt sich mit seinem distalen Kopplungsabschnitt 52 in die Axialbohrung 64, 65, 66, 67 der Adapterhülse 6. In der vergrößerten Darstellung von Fig. 4 und 5 ist zu sehen, dass der proximale Abschnitt 67 der Axialbohrung 64, 65, 66, 67 der Adapterhülse 6 sich kegelstumpfförmig zum proximalen Ende (in den Figuren nach rechts) aufweitet.

Dieser Konusabschnitt 67 ist passend zu einer konusförmigen Düse 21 einer Spülvorrichtung 20 geformt und dimensioniert, die in Fig. 5 angedeutet ist. Durch den zur konusförmigen Düse 21 passenden Konusabschnitt 67 wird bei Aufnahme der Düse 21 in dem Konusabschnitt 67 eine abgedichtete, klemmende Steckverbindung in der Art des "Luer-Slip"-Systems erreicht, die die axiale Zufuhr eines Spülmediums von proximaler Seite der Adaptervorrichtung 10 erlaubt. Das tritt in an dem Übertragungsadapter 5 vorbei die Axialbohrung 64, 65, 66, 67 der Adapterhülse 6 ein und gelangt von dort in den Schaft 2, dessen proximales Ende 2a in einem distalen Abschnitt 64 der Axialbohrung 64, 65, 66, 67 der Adapterhülse 6 aufgenommen ist.

Im vorliegenden Beispiel ist an dem proximalen Endabschnitt 63 der Adapterhülse 6 ein Außengewinde 63' ausgebildet, das zum Schraubeingriff mit einem Innengewinderohrabschnitt 21' der Spülvorrichtung 20 ausgebildet ist, der die Düse 21 umgibt. Die Schraubeingriff sichert die abdichtende Steckverbindung entsprechend dem "Luer-Lock"-System und gestattet daher auch höhere Drücke bei der Zufuhr des Spülmediums.

Zur Kopplung der Übertragungsstange 40 mit dem Übertragungsadapter 5 weist die Adaptervorrichtung 10 hier zwei Eingriffsschlitten 7 auf, wie auch in Fig. 3 zu sehen ist. Dazu sind an dem distalen Kopplungsabschnitt 52 des Übertragungsadapters 5 eine axiale Aufnahmebohrung 54 und zwei gegenüberliegende Eingriffsöffnungen 53 in Radialrichtung ausgebildet. Die Adapterhülse 6 weist dazu in einem taillierten mittleren Abschnitt 61 zwei gegenüberliegende Längsöffnungen 62 auf, die mit einem mittleren Abschnitt 66 der Axialbohrung 64, 65, 66, 67 verbunden sind.

Der distale Kopplungsabschnitt 52 des Übertragungsadapters 5 wird so in dem mittleren Abschnitt 66 der Adapterhülse 6 platziert, dass die Eingriffsöffnungen 53 am distalen Kopplungsabschnitt 52 passend zu den Längsöffnungen 62 an der Adapterhülse 6 ausgerichtet sind. Zur axialen Führung in Bezug auf die Adapterhülse 6 kann der distale Kopplungsabschnitt 52 des Übertragungsadapters 5 eine Vierkantform aufweisen.

Die von distaler Seite eingeführte Übertragungsstange 40 wird mit ihrem proximalen Kopplungselement 41 in die axiale Aufnahmebohrung 54 eingeführt, bis sich das Kopplungselement 41 im Bereich zwischen den Eingriffsöffnungen 53 befindet. Beide Eingriffsschlitten 7 werden mit ihrem jeweiligen Steckabschnitt 71 von beiden Seiten durch die jeweilige Längsöffnung 62 und die ausgerichtete Eingriffsöffnung 53 in die axiale Aufnahmebohrung 54 eingeführt, und nehmen das proximale Kopplungselement 41 der Übertragungsstange 40, wie in Fig. 4 zu sehen, formschlüssig in der jeweiligen Aussparung 72 des Steckabschnitts 71 auf. Die Längsöffnungen 62 sind länger als der Steckabschnitt 71, sodass mit dem Übertragungsadapter 5 verbundene Eingriffsschlitten 7 dessen translatorischer Bewegung relativ zur Adapterhülse 6 folgen kann. Für eine sichere Führung entsprich die Breite des Steckabschnitts 71 - bis auf das selbstverständlich für die Relativbewegung erforderliche Spiel - der Breite der Längsöffnung 62.

Im dargestellten Beispiel ist das proximale Kopplungselement 41 kugelkopfartig am proximalen Ende der Übertragungsstange 40 ausgebildet, sodass die Aussparung 72 entsprechend als Kugelkopfaufnahme geformt ist. Die Größe der Eingriffsöffnung 53 entspricht dem Querschnitt des Steckabschnitts 71, der damit formschlüssig in der Eingriffsöffnung 53 festgelegt ist. Um die Montage zu erleichtern, ist die axiale Aufnahmebohrung 54 so bemessen, dass das proximale Kopplungselement 41 zur einfachen Aufnahme in der Aussparung 72 des Steckabschnitts 71 nahe dem Bohrungsgrund platziert ist.

Um die Adapterhülse 6 im Bereich der Längsöffnungen 62 abzudichten, weist jeder Eingriffsschlitten 7 einen Schieberabschnitt 70 mit einer ebenen Innenseite auf, von der sich der Steckabschnitt 71 orthogonal erstreckt. Die Adapterhülse 6 ist an dem mittleren Abschnitt 61 um die Längsöffnung 62 mit einer ebenen Gleitfläche 68 ausgebildet, auf der der Schieberabschnitt 70 mit der ebenen Innenseite translatorisch bewegbar anliegt. Dabei sind Länge und Breite des Schieberabschnitts 70 so bemessen, dass die Längsöffnung 62 stets durch den Schieberabschnitt 70 abgedeckt ist, d. h. auch wenn der Steckabschnitt 71 am proximalen oder distalen Ende der Längsöffnung 62 anschlägt.

Um den Eingriff der Eingriffsschlitten 7 zu sichern, weisen diese an der Außenseite des Schieberabschnitts 70 im gezeigten Beispiel zwei in Umfangsrichtung verlaufende Aufnahmenuten 73 auf (vgl. Fig. 3), in denen jeweils ein elastischer O-Ring 8 aufgenommen ist, der, wie in Fig. 4 und 5 zu sehen den mittleren Abschnitt 61 der Adapterhülse 6 umgibt, um beide Eingriffsschlitten 7 zusammenzuhalten.

Die O-Ringe 8 ermöglichen eine Bewegung der Eingriffsschlitten 7 in Radialrichtung, die eine Verdrehung des Werkzeugeinsatzes mit dem Werkzeug 4 und der Übertragungsstange 40 während der Montage gestattet, z. B. zur Kopplung des Werkzeugs 4 an dem distalen Schaftende 2b mittels Bajonettverbindung. Im montierten Zustand des zerlegbaren Schaftinstruments 1 verhindert eine Aufnahmekontur im Gehäuseteil 30, wie in Fig. 2 angedeutet ist, dass sich die Eingriffsschlitten 7 anheben können. Somit ist es im montierten Zustand des zerlegbaren Schaftinstruments 1 bzw. während der Anwendung nicht möglich, den Werkzeugeinsatz zu demontieren.

Auch in demontierten Zustand des zerlegbaren Schaftinstruments 1, d. h. ohne Handhabungseinrichtung 3 und ohne den Werkzeugeinsatz aus Werkzeug 4 und Übertragungsstange 40, drücken die O-Ringe 8 die Eingriffsschlitten 7 auf die Gleitflächen 68 und verschließen damit die Längsöffnungen 62, sodass das Spülmedium proximal nur begrenzt austreten kann. Folglich wird das Spülmedium durch die Axialbohrung 64, 65, 66, 67 und durch den Schaft 2 bis an dessen distales Ende 2b weitergeleitet.

Die erfindungsgemäße Adaptervorrichtung, die ohne Gelenk-Feder-Verbindung auskommt, erlaubt die Konstruktion von medizinischen Schaftinstrumenten mit axialer Zufuhr eines Spülmediums für minimalinvasive Instrumente, die insbesondere auch Schaftdurchmesser von weniger als 5 mm aufweisen können. Dabei ist eine optimierte Durchspülung im proximalen Bereich der eingesetzten Adaptervorrichtung ohne zusätzlichen Spüladapter oder andere Bauteile möglich, da die Adaptervorrichtung durch ihre Konstruktion selbst abgedichtet ist. Durch Integration des Spülanschlusses in den proximalen Endabschnitt der Adapterhülse kann die Aufbereitung der zerlegbaren Schaftinstrumente optimiert werden. Der einfache Aufbau der Adaptervorrichtung mit wenigen Bauteilen und einfachen Konturen ohne Hinterschnitte sowie wenigen abgedeckten Flächen erlaubt außerdem den Verzicht auf dauerhafte Verbindungstechniken wie Schweißen oder Kleben oder zumindest deren Minimierung.

Die vorliegende Erfindung stellt eine Adaptervorrichtung und ein damit ausgestattetes zerlegbares medizinisches Schaftinstrument, ein Verfahren zum Reinigen des zerlegbaren medizinischen Schaftinstruments und ein Verfahren zum Montieren der Adaptervorrichtung zur Bereitstellung des zerlegbaren medizinischen Schaftinstruments bereit. Die Adaptervorrichtung 10 ist zur Anordnung in einem zerlegbaren medizinischen Schaftinstrument 1 mit einem Schaft 2, einer im Schaft 2 bewegbaren Übertragungsstange 40 und einer Handhabungseinrichtung 3 mit einem Gehäuseteil 30 und einem Betätigungsteil 31, das gegenüber dem Gehäuseteil 30 bewegbar ist, ausgebildet. Die Adaptervorrichtung 10 hat einen Übertragungsadapter 5, der zur lösbaren Kopplung mit dem Betätigungsteil 31 und mit der Übertragungsstange 40 ausgebildet ist, und eine Adapterhülse 6, die zur lösbaren Kopplung mit dem Schaft 2 und mit dem Gehäuseteil 30 ausgebildet ist. Die Adapterhülse 6 hat eine Axialbohrung 64, 65, 66, 67, in der ein distaler Kopplungsabschnitt 52 des Übertragungsadapters 5 angeordnet ist, und wobei der Übertragungsadapter 5 relativ zur Adapterhülse 6 translatorisch bewegbar ist. Erfindungsgemäß ist ein proximaler Abschnitt 67 der Axialbohrung 64, 65, 66, 67 der Adapterhülse 6 kegelstumpfförmig ausgebildet und weitet sich zum proximalen Ende der Axialbohrung 64, 65, 66, 67 auf, wobei der kegelstumpfförmige proximale Abschnitt 67 zur lösbaren Aufnahme einer konusförmigen Düse 21 einer Spülvorrichtung 20 ausgebildet ist. Die Zeichnungen, die Beschreibung und die Ansprüche enthalten zahlreiche Merkmale in Kombination. Es versteht sich, dass die vorstehend genannten Merkmale nicht nur in der jeweils angegebenen Kombination, sondern auch in anderen Kombinationen oder in Alleinstellung verwendbar sind, ohne den Rahmen der vorliegenden Erfindung zu verlassen.

### BEZUGSZEICHENLISTE

- 1, 1': Zerlegbares Schaftinstrument
- 2: Schaft
- 2a, 2b: proximales, distales (Schaft-) Ende
- 3: Handhabungseinrichtung
- 4: Werkzeug
- 5, 5': Übertragungsadapter
- 6: Adapterhülse
- 7: Eingriffsschlitten
- 8: O-Ring zur Sicherung
- 9: O-Ring zur Abdichtung
- 10: Adaptervorrichtung
- 11: Spülanschluss nach dem Stand der Technik
- 20, 21: Spülvorrichtung, konusförmige Düse
- 30, 31, 32: Gehäuseteil, Betätigungsteil, Deckelelement
- 40, 41: Übertragungsstange, proximales Kopplungselement/Kugelkopf
- 50, 51: Stangenabschnitt, proximales Kopplungselement/Kugelkopf
- 52: Distaler Kopplungsabschnitt
- 53: Eingriffsöffnung
- 54: Aufnahmebohrung
- 60, 60': distaler Endabschnitt, Wellenbund
- 61: Mittlerer Abschnitt
- 62: Längsöffnung
- 63, 63': proximaler Endabschnitt, Außengewinde
- 64: Distaler Abschnitt der Axialbohrung
- 65, 65': Verbindungsabschnitt der Axialbohrung, Dichtnut
- 66: Mittlerer Abschnitt der Axialbohrung
- 67: Proximaler Konusabschnitt der Axialbohrung/
- 68: Gleitfläche
- 70: Schieberabschnitt
- 71: Steckabschnitt
- 72: Aussparung
- 73: Aufnahmenut

## Patentansprüche

1. Adaptervorrichtung (10), ausgebildet zur Anordnung in einem zerlegbaren medizinischen Schaftinstrument (1) mit einem Schaft (2), einer im Schaft (2) bewegbaren Übertragungsstange (40) und einer Handhabungseinrichtung (3) mit einem Gehäuseteil (30) und einem Betätigungsteil (31), das gegenüber dem Gehäuseteil (30) bewegbar ist, wobei die Adaptervorrichtung (10) aufweist:
- einen Übertragungsadapter (5), der zur lösbaren Kopplung mit dem Betätigungsteil (31) und mit der Übertragungsstange (40) ausgebildet ist, und
- eine Adapterhülse (6), die zur lösbaren oder unlösbaren Kopplung mit dem Schaft (2) und mit dem Gehäuseteil (30) ausgebildet ist,
wobei die Adapterhülse (6) eine Axialbohrung (64, 65, 66, 67) aufweist, in der ein distaler Kopplungsabschnitt (52) des Übertragungsadapters (5) angeordnet ist, und wobei der Übertragungsadapter (5) relativ zur Adapterhülse (6) translatorisch bewegbar ist,
**dadurch gekennzeichnet, dass**
ein proximaler Abschnitt (67) der Axialbohrung (64, 65, 66, 67) der Adapterhülse (6) kegelstumpfförmig ausgebildet ist und sich zum proximalen Ende der Axialbohrung (64, 65, 66, 67) aufweitet, wobei der kegelstumpfförmige proximale Abschnitt (67) zur lösbaren Aufnahme einer konusförmigen Düse (21) einer Spülvorrichtung (20) ausgebildet ist.

2. Adaptervorrichtung (10) nach Anspruch 1,
**dadurch gekennzeichnet, dass**
ein proximaler Endabschnitt (63) der Adapterhülse (6) ein Außengewinde (63') aufweist, das zur lösbaren Verbindung mit einem die Düse (21) umgebenden Innengewinderohrabschnitt (21') der Spülvorrichtung (20) ausgebildet ist.

3. Adaptervorrichtung (10) nach Anspruch 1 oder 2,
**dadurch gekennzeichnet, dass**
der proximale kegelstumpfförmige proximale Abschnitt (67) entsprechend einem genormten Verbindungssystem geformt ist.

4. Adaptervorrichtung (10) nach zumindest einem der Ansprüche 1 bis 3,
**dadurch gekennzeichnet, dass**
der distale Kopplungsabschnitt (52) des Übertragungsadapters (5) eine axiale Aufnahmebohrung (54) und zumindest eine damit verbundene Eingriffsöffnung (53) in Radialrichtung aufweist, und
die Adaptervorrichtung (10) zumindest einen Eingriffsschlitten (7) mit einem Steckabschnitt (71) aufweist, der in der Eingriffsöffnung (53) angeordnet ist und sich in die Aufnahmebohrung (54) des Übertragungsadapters (5) erstreckt und eine Aussparung (72) aufweist, die zur Aufnahme eines proximalen Kopplungselements (41) der Übertragungsstange (40) ausgebildet ist,
wobei die Adapterhülse (6) an einem mittleren Abschnitt (61) zumindest eine Längsöffnung (62) aufweist, die mit der Axialbohrung (64, 65, 66, 67) verbunden ist und länger ist als der Steckabschnitt (71), der sich durch die Längsöffnung (62) zur Anordnung in der Eingriffsöffnung (53) des distalen Kopplungsabschnitts (52) erstreckt, wobei der Eingriffsschlitten (7) mit dem Übertragungsadapter (5) relativ zur Adapterhülse (6) translatorisch bewegbar ist.

5. Adaptervorrichtung (10) nach Anspruch 4,
**dadurch gekennzeichnet, dass**
der Eingriffsschlitten (7) einen Schieberabschnitt (70) aufweist, von dem sich der Steckabschnitt (71) erstreckt, wobei die Adapterhülse (6) an dem mittleren Abschnitt (61) um die Längsöffnung (62) eine Gleitfläche (68) aufweist, auf der der Schieberabschnitt (70) des Eingriffsschlittens (7) translatorisch bewegbar anliegt, wobei eine Länge und Breite des Schieberabschnitts (70) so bemessen sind, dass die Längsöffnung (62) stets durch den Schieberabschnitt (70) abgedeckt ist.

6. Adaptervorrichtung (10) nach Anspruch 4 oder 5,
**dadurch gekennzeichnet, dass**
die Adaptervorrichtung (10) zwei Eingriffsschlitten (7), zwei Eingriffsöffnungen (53) an dem distalen Kopplungsabschnitt (52) des Übertragungsadapters (5) und zwei Längsöffnungen (62) an dem mittleren Abschnitt (61) der Adapterhülse (6) aufweist, die jeweils in Radialrichtung gegenüberliegend angeordnet sind.

7. Adaptervorrichtung (10) nach zumindest einem der Ansprüche 4 bis 6,
**dadurch gekennzeichnet, dass**
der Eingriffsschlitten (7) an einer von dem Steckabschnitt (71) abgewandten Außenseite zumindest eine in Umfangsrichtung verlaufende Aufnahmenut (73) aufweist, in der ein elastischer O-Ring (8) aufgenommen ist, der den mittleren Abschnitt (61) der Adapterhülse (6) umgibt.

8. Adaptervorrichtung (10) nach zumindest einem der Ansprüche 1 bis 7,
**dadurch gekennzeichnet, dass**
der distale Kopplungsabschnitt (52) zur axialen Führung des Übertragungsadapters (5) in der Adapterhülse (6) eine Kantform mit zumindest einer axialparallelen Führungsfläche aufweist.

9. Adaptervorrichtung (10) nach zumindest einem der Ansprüche 1 bis 8,
**dadurch gekennzeichnet, dass**
ein distaler Abschnitt (64) der Axialbohrung (64, 65, 66, 67) zur lösbaren oder unlösbaren Aufnahme eines proximalen Endes (2a) des Schafts (2) ausgebildet und mit einem im Durchmesser reduzierten Verbindungsabschnitt (65) der Axialbohrung (64, 65, 66, 67) verbunden ist, der zum Durchtritt der Übertragungsstange (40) ausgebildet ist,
und/oder
ein distaler Endabschnitt (60) der Adapterhülse (6) zylindrisch geformt ist und durch einen Wellenbund (60') begrenzt wird, der als Anschlag zur Anordnung in dem Gehäuseteil (30) ausgebildet ist.

10. Zerlegbares medizinisches Schaftinstrument (1), das eine Handhabungseinrichtung (3) mit einem Gehäuseteil (30) und einem relativ dazu bewegbaren Betätigungsteil (31), einen Schaft (2), eine im Schaft (2) bewegbare Übertragungsstange (40) und eine Adaptervorrichtung (10) aufweist, die einen Übertragungsadapter (5) zur lösbaren Kopplung mit dem Betätigungsteil (31) und mit der Übertragungsstange (40) und eine Adapterhülse (6) zur lösbaren oder unlösbaren Kopplung mit dem Gehäuseteil (30) und mit dem Schaft (2) aufweist,
wobei ein distaler Kopplungsabschnitt (52) des Übertragungsadapters (5) in einer Axialbohrung (64, 65, 66, 67) der Adapterhülse (6) angeordnet ist, und der Übertragungsadapter (5) relativ zur Adapterhülse (6) translatorisch bewegbar ist, **dadurch gekennzeichnet, dass**
die Adaptervorrichtung (10) eine Adaptervorrichtung (10) nach zumindest einem der Ansprüche 1 bis 9 ist.

11. Verfahren zum Reinigen eines zerlegbaren medizinischen Schaftinstruments (1) nach Anspruch 10,
umfassend die Schritte
- Lösen der Kopplung des Übertragungsadapters (5) mit dem Betätigungsteil (31) und Lösen der Kopplung der Adapterhülse (6) von dem Gehäuseteil (30), und
- Trennen des mit der Übertragungsstange (40) gekoppelten Übertragungsadapters (5) und insbesondere der mit dem Schaft (2) gekoppelten Adapterhülse (6) aus der Handhabungseinrichtung (3),
- Lösen der Kopplung des Übertragungsadapters (5) mit der Übertragungsstange (40) und Entfernen der Übertragungsstange (40) aus dem Schaft (2),
- Anordnen der konusförmigen Düse (21) einer Spülvorrichtung (20) in dem kegelstumpfförmigen proximalen Abschnitt (67) der Axialbohrung (64, 65, 66, 67) der Adapterhülse (6) und
- Zuführen eines Spülmediums mit der Spülvorrichtung (20) durch die Düse (21) in die Axialbohrung (64, 65, 66, 67) der Adapterhülse (6) und den damit gekoppelten Schaft (2), dadurch Reinigen des medizinischen Schaftinstruments (1).

12. Verfahren zum Montieren einer Adaptervorrichtung (10) nach zumindest einem der Ansprüche 1 bis 9 zur Bereitstellung eines zerlegbaren medizinischen Schaftinstruments (1) nach Anspruch 10,
umfassend die Schritte
- Bereitstellen des Übertragungsadapters (5) und der Adapterhülse (6) und Bereitstellen der Handhabungseinrichtung (3) mit dem Gehäuseteil (30) und dem relativ dazu bewegbaren Betätigungsteil (31) und dem Schafts (2) mit der im Schaft (2) bewegbaren Übertragungsstange (40),
- Anordnen des distaler Kopplungsabschnitts (52) des Übertragungsadapters (5) in der Axialbohrung (64, 65, 66, 67) der Adapterhülse (6),
- lösbares Koppeln des Übertragungsadapters (5) mit der Übertragungsstange (40) und lösbar oder unlösbares Koppeln der Adapterhülse (6) mit dem Schaft (2),
- Anordnen des mit der Übertragungsstange (40) gekoppelten Übertragungsadapters (5) und der mit dem Schaft (2) gekoppelten Adapterhülse (6) in der Handhabungseinrichtung (3), dabei
- lösbares Koppeln der Adapterhülse (6) mit dem Gehäuseteil (30) und lösbares Koppeln des Übertragungsadapters (5) mit dem Betätigungsteil (31).

13. Verfahren nach Anspruch 12, wobei das lösbare Koppeln des Übertragungsadapters (5) mit der Übertragungsstange (40) ferner die Schritte umfasst:
- beim Anordnen des distaler Kopplungsabschnitts (52) des Übertragungsadapters (5) in der Axialbohrung (64, 65, 66, 67) der Adapterhülse (6) Ausrichten der zumindest einen mit der axialen Aufnahmebohrung (54) verbundenen Eingriffsöffnung (53) am distalen Kopplungsabschnitt (52) mit der zumindest einen Längsöffnung (62) an dem mittleren Abschnitt (61) der Adapterhülse (6),
- Anordnen des proximalen Kopplungselements (41) der Übertragungsstange (40) in der Aufnahmebohrung (54) am distalen Kopplungsabschnitt (52),
- Bereitstellen des zumindest eines Eingriffsschlittens (7),
- Einführen des Steckabschnitts (71) des Eingriffsschlittens (7) durch die Längsöffnung (62) und die Eingriffsöffnung (53) in die Aufnahmebohrung (54) und
- Aufnehmen des proximalen Kopplungselements (41) der Übertragungsstange (40) in der Aussparung (72) des Steckabschnitts (71).

14. Verfahren nach Anspruch 13, wobei das lösbare Koppeln des Übertragungsadapters (5) mit der Übertragungsstange (40) ferner den Schritt umfasst:
- Anordnen eines elastischen O-Rings (8) in der Aufnahmenut (73) an der Außenseite des Eingriffsschlittens (7).
